# EUROPEAN PATENT APPLICATION

(11) **EP 2 034 749 A2**
(43) Date of publication of application: **11.03.2009**
(21) Application number: 08101372.4
(22) Date of filing: 07.02.2008
(51) Int. Cl.: H04Q 3/00

(54) **Method for chaining services in a telecommunication network and a service control point comprising means for implementing this method**

(30) Priority: 10.09.2007 IN CH20272007
(71) Applicant: Alcatel Lucent, 75008 Paris (FR)
(72) Inventor: Thakur, Jitendra, 462016 Madhya Pradesh (IN); Goel, Neena, 110088 Delhi (IN)
(74) Representative: Sciaux, Edmond

(57) **Abstract**

A method is disclosed of chaining services in a telecommunication network comprising at least one service switching point (1') and at least one service control point (2'), this latter comprising means for providing a plurality of services (3', 4', 5'). The services at service control point can be chained by adding a listener service (6') at service control point, the listener service will trigger different services (3', 4', 5') if configured. The chained services will receive Initial DP message not from service switching point but from listener service. There is no customization required different chained services(3', 4', 5'). These services can receive Initial message from network as well as from listener service

## Description

### BACKGROUND OF THE INVENTION

### Field of the invention

The present invention generally relates to a method of chaining services in a telecommunication network, to provide a optimized and cost effective solution and to avoid loop back between Service Switching Point (SSP) and Service Control point (SCP).

In a telecommunication network comprising a Service Switching Point (SSP) and a Service Control point (SCP), the services are provided in the Service Control point (SCP) and each service is requested by sending a signaling message from the SSP to the SCP, and the request is accepted or rejected by sending back a response message.

Traditional way of service chaining is to do loop back between the SSP and the SCP, once for each service, so that for the same call at least another service can be triggered. This way implies exchanging a lot of signaling messages between the SSP and the SCP.

The aim of the invention is to increase network efficiency by supporting service chaining with less numerous signaling messages between the SSP and a SCP and thus providing a cost effective and time saving solution.

### Description of the prior art

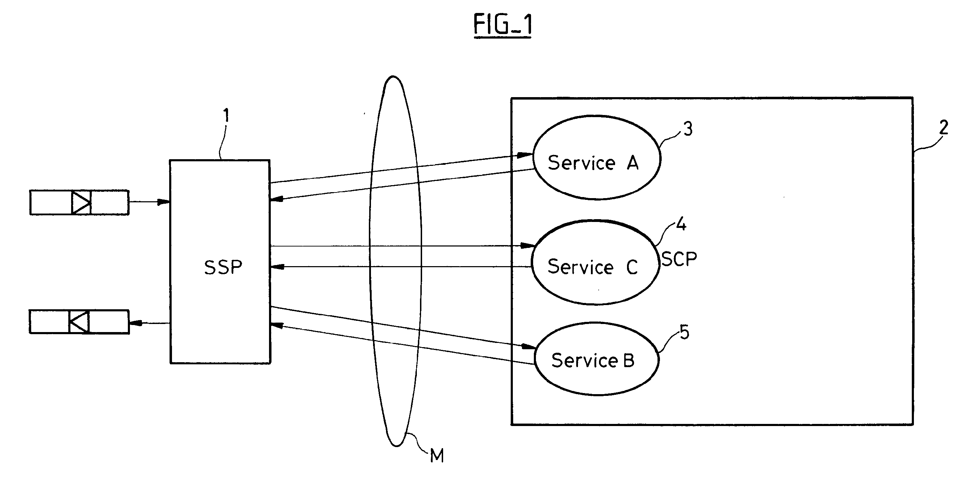

**Figure 1** shows one example of a classical telecommunication network comprising a Service Switching Point (SSP) 1 and a classical Service Control point (SCP) 2. Three services 3, 4, 5 are provided in the SCP 2 and each service is requested by sending a signaling message from the SSP 1 to the SCP 2, and the request for a service is accepted or rejected by sending back a response message.

For chaining these three services 3, 4, 5, the SSP 1 triggers SCP 2 three times before sending any Continue/Connect message to another party. For service chaining, currently loop back is done at SSP 1. This way implies exchanging a lot of signaling messages M, between the SSP 1 and the SCP 2, thus resulting in network traffic, time loss and also expense.

### SUMMARY OF THE INVENTION

The invention provides a method of chaining services in a telecommunication network comprising at least one service switching point and at least one service control point, this latter comprising means for providing a plurality of services ; characterized in that it further comprises means for providing a listener service which can trigger a plurality of chained services chosen among the services implemented in the service control point.

According to this method, each service can be triggered independently by network or can be chained within SCP. This will save signaling messages between SSP and SCP.

According to another aspect of the present invention, there is provided a service control point comprising means for implementing this method .

Other features and advantages of the present invention will become more apparent from the following detailed description of an embodiment of the present invention, when taken in conjunction with the accompanying drawings.

### BRIEF DESCRIPTION OF THE DRAWINGS

In order to illustrate in detail features and advantages of an embodiment of the present invention, the following will be with reference to the accompanying drawings. If possible, like or similar reference numerals designate the same or similar components throughout the figures thereof and description, in which :
- Fig. 1 illustrates the prior art method, and has been described above.
- Fig. 2 illustrates the method according to the invention.

### DETAILED DESCRIPTION OF A PREFERRED EMBODIMENT

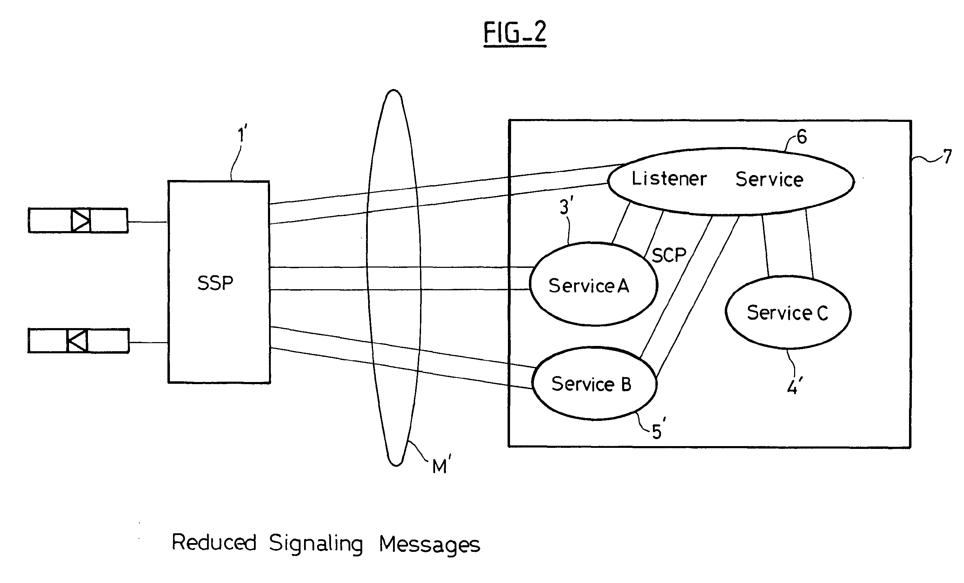

**Figure 2** shows one example of a telecommunication network comprising a Service Switching Point (SSP) 1' and a Service Control point (SCP) 7 comprising means for implementing the method according to the invention. Three services 3', 4', 5' are provided in the SCP 7 and each service can be requested individually by sending a signaling message from the SSP 1' to the SCP 7, and the request for a service is accepted or rejected by sending back a response message.

For chaining two or three of these three services 3', 4', 5', a fourth service 6 is provided in the SCP 7, the listener service 6 triggering a plurality of chained services chosen among the services 3', 4', 5' provided in the SCP 7.

For instance, the SSP 1' requests that the three services 3', 4', 5' (for instance Black and White, Number Portability and Optimal Call Routing) be chained. The chaining order and choice of services to be chained are controlled by service key. The fourth service 6 is working as a listener service which listens to initial message from SSP 1' and sends Initial Detection Point (DP) with Transaction Capabilities Application Part (TCAP) header to rest of the services 3', 4', 5'. A Detection Point indicates states in basic call and connection over F-Dpe processing where control may be transferred to the service. Transaction Capabilities Application Part enables the deployment of advanced intelligence in a network by supporting non circuit related information exchange between signaling points, using the SSCP (Signaling Connection Control Part) connectionless service.

There is no customization required for different chained services. These services can receive Initial message from network as well as from listener service.

The method according to the invention has many advantages as follows: As different services are triggered at SCP itself, no signaling message is exchanged with SSP for service triggering. It results in reduced signaling messages, which in turn means reduced signaling link requirement and reduced signaling cards. It results a reduced cost of equipment.

The inter service triggering message will be on a F-Dpe or a Gigabit local area network, in the SCP, which is obviously very fast as compared to a 64 kp/s signaling link.

There is no customization required for different chained services. These services can receive Initial message from network as well as from listener service.

An advantage of the method according to the invention lies in the fact that, for each call, it can be configured which all services will be chained and in which particular sequence. The sequence of services to be chained can be configured at SSP either by keeping different service keys, each key referring to one particular sequence at SCP, or by provisioning different Service Switch Nodes. This method of service chaining can be implemented on any SCP supporting multiple services.

## Claims

1. A method of chaining services in a telecommunication network comprising at least one service switching point (1') and at least one service control point (2'), this latter comprising means for providing a plurality of services (3', 4', 5') ; **characterized in that** it further comprises means for providing a listener service (6) which can trigger a plurality of chained services chosen among the services (3', 4', 5') implemented in the service control point (2').

2. A service control point (2') comprising means for implementing the method according to claim 1.
